(19) Europäisches Patentamt
European Patent Office
Office européen des brevets

(11) EP 0 469 975 B1

(12) EUROPEAN PATENT SPECIFICATION

(45) Date of publication and mention of the grant of the patent:
**14.08.1996 Bulletin 1996/33**

(51) Int Cl.6: **C09K 19/32**

(21) Application number: **91402104.3**

(22) Date of filing: **26.07.1991**

(54) **Liquid crystal compound**

Flüssigkristalline Verbindung

Composé cristal liquide

(84) Designated Contracting States:
**DE FR GB**

(30) Priority: **30.07.1990 JP 201607/90**

(43) Date of publication of application:
**05.02.1992 Bulletin 1992/06**

(73) Proprietor: **Showa Shell Sekiyu Kabushiki Kaisha Tokyo (JP)**

(72) Inventors:
- **Aihara, Yoshihiko, c/o Showa Shell Sekiyu K.K. Chiyoda-ku, Tokyo 100 (JP)**
- **Isozaki, Tadaaki, c/o Showa Shell Sekiyu K.K. Chiyoda-ku, Tokyo 100 (JP)**
- **Yamakawa, Noriko, c/o Showa Shell Sekiyu K.K. Chiyoda-ku, Tokyo 100 (JP)**
- **Kawamura, Ichiro, c/o Showa Shell Sekiyu K.K. Chiyoda-ku, Tokyo 100 (JP)**

(74) Representative: **Eidelsberg, Victor Albert et al**
**Cabinet Flechner**
**22, Avenue de Friedland**
**75008 Paris (FR)**

(56) References cited:
**EP-A- 0 339 987**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 469 975 B1

Printed by Jouve, 75001 PARIS (FR)

## Description

The present invention relates to ferroelectric chiral smectic liquid crystal compounds which are used for display devices or electrooptical devices utilizing responses to electric fields.

Furthermore, the present invention relates to antiferroelectric liquid crystal compounds which show three stable molecular orientations optically tristable states. The liquid crystal compounds are used for display devices or electrooptical devices utilizing response to electric fields.

As electrooptical devices utilizing liquid crystals, those which use nematic liquid crystals such as DSM type, TN type, G-H type, and STN type have been developed and practically used. However, their use is restricted because of their slow response time speed as slow as a few millisec to some ten millisec. The slow response speed of devices which use nematic liquid crystals is due to the fact that torque for moving molecules is not so large since the torque is based on anisotropy of dielectric constant. Under such circumstances, Meyer et al. developed ferroelectric liquid crystals which have spontaneous polarization (Ps), and have a large torque (Ps x E where E is an applied electric field). Thus they have a high response speed of a few μsec to some ten μsec. (Meyer et al, Le Journal de Physique, 36, 1975, L-69). Another novel antiferroelectric liquid crystal is disclosed in Japanese Patent Kokai No. 63-307837. The term "tristable states" referred to hereinafter is disclosed in Japanese Patent Kokai Nos. 1-316367, 1-316372, 1-316339, 2-28128, 2-131450, 2-160748 and No. 1-213390.

Some high speed electrooptical devices utilizing ferroelectric liquid crystals have been proposed.

A typical example is a device where a helical structure is released by wall forces and change is brought about in two molecular orientations which become parallel to the wall by polarity of applied fields (Japanese Patent Kokai No. 56-107216).

The above one is based on a premise that there is a compound having an ideal bistable states as shown in response wave to electric field of Fig. 1. However, such compound which has the ideal bistable states has not yet been found. Response wave to electric field of bistable state liquid crystals which have been actually synthesized is as shown in Fig. 2 and the response wave as shown in Fig. 1 has not been obtained. When such liquid crystal having the response wave as shown in Fig. 2 is used, for example, for switching circuits for light, the object cannot be sufficiently attained by only a change in applied electric voltage between "ON" and "OFF", since percent transmission gradually changes as applied electric voltage changes from ⊖ side to ⊕ side. Moreover, it is difficult for the bistable state liquid crystals which have been hitherto synthesized to form a monodomain structure which is an ideal molecular orientation at an S*c phase stage where no electric field is applied to and disclination (defect) is formed or a disturbance called twist is caused in molecular orientation. Thus, it is difficult to realize the ideal bistable state orientation with large area. Furthermore, since threshold value (voltage at which luminance varies by a given value) is small, contrast decreases or field of view narrows when dynamic driving is applied. Besides, the bistable state liquid crystals which have been actually synthesized cannot show the hysteresis as shown in Fig. 1, but shows only the hysteresis as shown in Fig. 2 and so they have no memory effect. Therefore, a large amount of energy is lost in order for the liquid crystals keeping a stable response at the S*c phase, since the voltage $v_3$ in Fig. 2 must be continuously applied or a high frequency must be continuously applied.

Although high speed liquid crystal electrooptical devices which utilize effectively the strong bonding between applied electric field and molecular orientation of ferroelectric liquid crystals are desired, there are still many problems to be solved.

Therefore, the object of the present invention is to provide novel liquid crystal compounds which can be used for liquid crystal electrooptical devices utilizing the tristable states to make high speed response possible where stable molecular orientations of clear occulating contrast are realized depending on electric fields applied, clear threshold specificity and clear hysteresis as shown in Fig. 3 are revealed, and dynamic driving can be easily realized.

In the present invention, novel antiferroelectric liquid crystals are provided which have utterly novel tristable states different from chiral smectic C phase (S*c phase) which is conventional bistable state phase.

The term "having tristable states" means that an electrooptical device where antiferroelectric liquid crystals are laid between the first electrode substrate plate and the second electrode substrate plate which is apart at a given space from the first one is constructed. A triangular electric voltage is applied to both the first and second electrode substrate plates as shown in Fig. 4 A. The antiferroelectric liquid crystals have molecular orientation of the first stable state (as shown in Fig. 4 D-2) where no electric field is applied, but upon application of electric field, molecular orientation of the second stable state (as shown in Fig. 4 D-1) which is different from the first stable state in one of electric field directions and molecular orientation of the third stable state (as shown in Fig. 4 D-3) which is different from the first and second stable states in the other direction of electric field. Liquid crystal electrooptical devices utilizing this tristable states are disclosed in Japanese Patent Kokai No. 2-153322.

On the other hand, "commercially available nematic liquid crystals" or bistable state liquid crystals do not have the three stable states as shown in Fig. 4 B and C.

The novel tristable state antiferroelectric liquid crystals exhibit epochmaking effects when they are used in liquid

crystal devices as compared with conventional nematic liquid crystals.

For obtaining high image quality, the conventional liquid crystals require much complicated structure driven by active matrix system while the tristable state antiferroelectric liquid crystals require simple matrix displays. Therefore the high production cost and, complicated, fabrication processes of the conventional liquid crystal device restrict their scope for large scale production, while for the tristable state antiferroelectric liquid crystals, production processes are simple even for large scale displays. As the result the production cost is relatively low.

Fig. 1 shows hysteresis of ideal bistable state liquid crystal which has not been actually obtained.

Fig. 2 shows hysteresis of bistable state liquid crystal which has been actually synthesized.

Fig. 3 shows hysteresis of the tristable state liquid crystal of the present invention.

In Figs. 1-3, abscissa axis shows applied voltage and ordinate axis shows transmittance (%).

Fig. 4A shows applied triangular wave, Figs. 4B, 4C and 4D show optical response of commercially available nematic liquid crystal, that of bistable state liquid crystal, and that of tristable state liquid crystal, respectively.

Figs. 5 and 6 show IR spectra of the liquid crystal compounds of Examples 1 and 2.

The first claim of the present invention relates to a liquid crystal compound which is represented by the formula [I]:

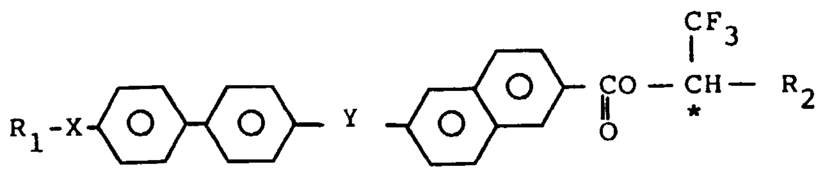 ... [I]

, wherein $R_1$ represents an alkyl group of 5 - 18 carbon atoms; $R_2$ represents an alkyl group of 6-16 carbon atoms; X represents a -O-,

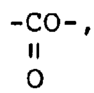

Y represents

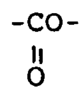 or 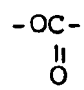

and when X is O Y is

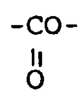

; and * indicates an optically active center.

The second claim of the present invention relates to a liquid crystal compound which is represented by the formula [II]:

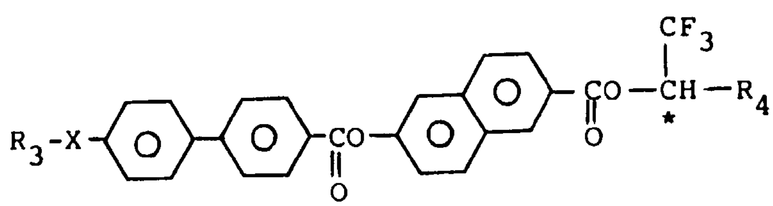 ... [II]

, wherein $R_3$ represents an alkyl group of 8 - 18 carbon atoms; $R_4$ represents an alkyl group of 6-14 carbon atoms; X represents a -O-,

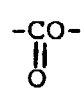

and * indicates an optically active center.

The liquid crystal compounds of the present invention can be used for liquid crystal electrooptical devices utilizing optically tristable states and besides, can be used for liquid crystal electrooptical devices utilizing conventional optically bistable state liquid crystals.

Some synthesis examples of the present compounds are shown below.

(A)

(i) 2-Hydroxy-6-carboxynaphthalene is allowed to react with an optically active 1-trifluoromethylalkyl alcohol in ethylene chloride in the presence of sulfuric acid to obtain l-trifluoromethylalkyl 6-hydroxynaphthalene-2-carboxylate.

(ii) The reaction product is allowed to react with 4-n-alkyloxy-4'-carboxybiphenyl in the presence of dicyclohexylcarbodiimide to obtain 1-trifluoromethylalkyl 6-[4-(n-alkyloxy)biphenyl-4'-carbonyloxy]naphthalene-2-carboxylate.

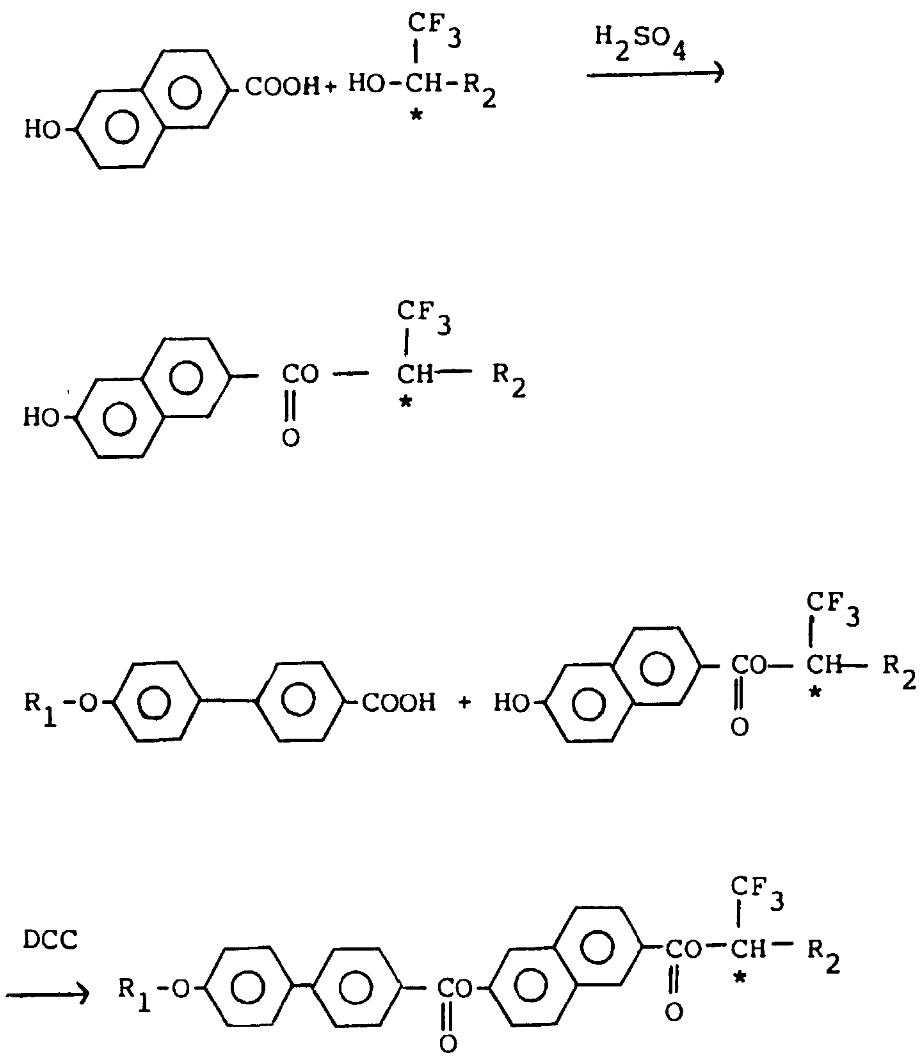


DCC: Dicyclohexylcarbodiimide

(B)

(i) 2,6-Naphthalenedicarbonyl dichloride is allowed to react with optically active 1,1,1-trifluoro-2-alkanol to obtain 6-(1,1,1-trifluoro-2-alkyloxycarbonyl)naphthalene-2-carbonyl chloride.

(ii) A fatty acid chloride is allowed to react with 4-methylcarbonyloxy-4'-hydroxybiphenyl and the reaction product is hydrolyzed with tert-butylamine to obtain 4-n-alkanoyloxy-4'-hydroxybiphenyl.

(iii) The above product is allowed to react with 6-(1,1,1-trifluoro-2-alkyloxycarbonyl)-naphthalene-2-carboxylic acid chloride in the presence of triethylamine to obtain 1,1,1-trifluoro-2-alkyl 6-[4-(n-alkanoyldxy)biphenyl-4'-oxycarbonyl]naphthalene-2-carboxylate.

(i)

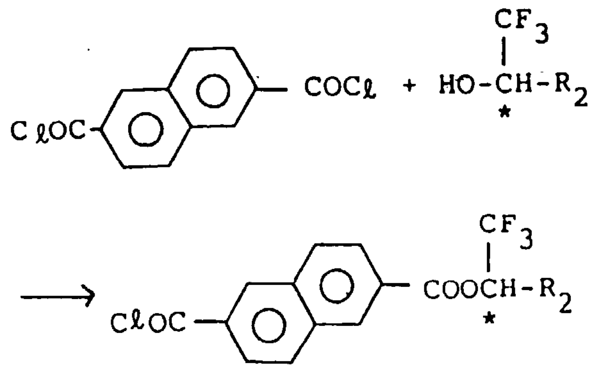

(ii)

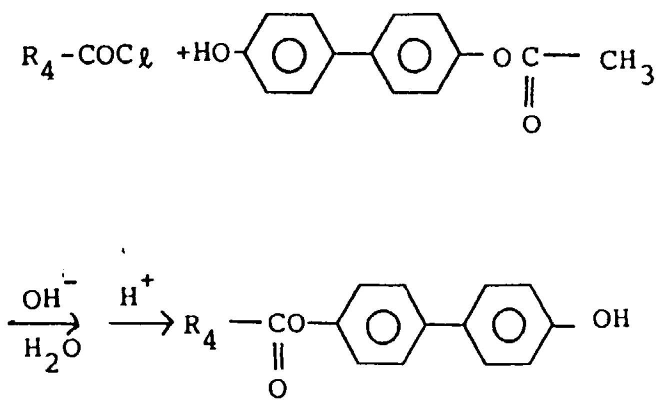

(iii)

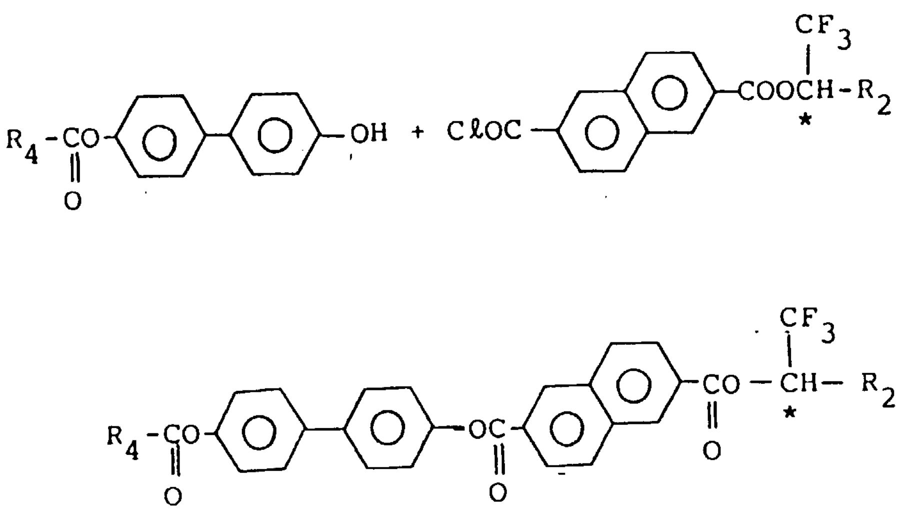

The present invention will be explained by the following examples, which should not be construed as limiting the invention.

Example 1 (process A)

(1) Synthesis of 1-trifluoromethylheptyl 6-hydroxynaphthalene-2-carboxylate

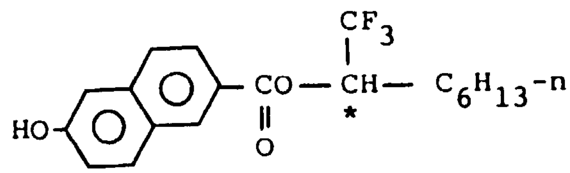

To a solution of 2-hydroxy-6-carboxylnaphthalene (2 g) and optically active 1-trifluoromethylheptyl alcohol (2.2 g) in ethylene chloride (50 ml) were added a few drops of concentrated sulfuric acid and the solution was refluxed with stirring for about 2 days. The reaction mixture was poured in water and the organic layer was collected. This was washed with aqueous sodium hydroxide solution and then with water and dried over anhydrous magnesium sulfate and then, the solvent was distilled off. The residue was purified by silica gel column chromatography (developer: chloroform) to obtain the titled compound (1.2 g).

(2) Synthesis of 1-trifluoromethylheptyl 6-[4-(n-octyloxy)biphenyl-4'-carbonyloxy]-naphthalene-2-carboxylate

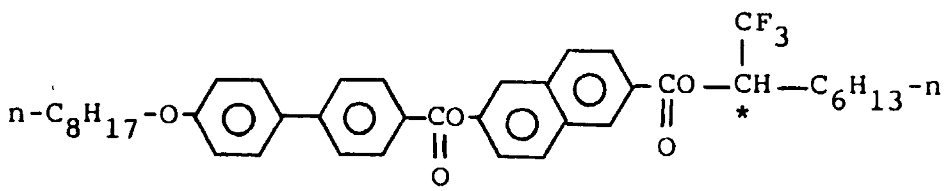

To a solution of 1-trifluoromethylheptyl 6-hydroxynaphthalene-2-carboxylate (0.6 g) obtained in (1) above and 4-n-octyloxy-4'-carboxylbiphenyl (0.5 g) in tetrahydrofuran (40 ml) were added dicyclohexylcarbodiimide (0.5 g) and dimethylaminopyridine (0.07 g), and the mixture was stirred for 24 hours.

After the solvent was distilled off, the residue was dissolved in dichloromethane (40 ml). The solution was washed with dilute hydrochloric acid and then with water and dehydrated with anhydrous magnesium sulfate. The solvent was distilled off and the residue was purified by silica gel column chromatography (developer: hexane/ethyl acetate = 20/1) to obtain the titled compound (0.42 g) having specific rotation $[\alpha]_D^{20}$ = +41.6°.

Phase transition temperatures (°C) which were observed under a polarizing microscope using a hot stage were as follows.

$$\text{Cry} \xleftarrow[93.5]{} \text{S*(3)} \xleftarrow[147.4]{} \text{S*c} \xleftarrow[150.7]{} \text{SA} \xleftarrow[176.4]{} \text{Iso}$$

wherein S*(3): optically tristable state liquid crystal phase.

IR spectrum (KBr) of the titled compound is shown in Fig. 5.

Example 2 (process A)

Synthesis of 1-trifluoromethylnonyl 6-[4-(n-octyloxy)biphenyl-4'-carbonyloxy]-naphthalene-2-carboxylate

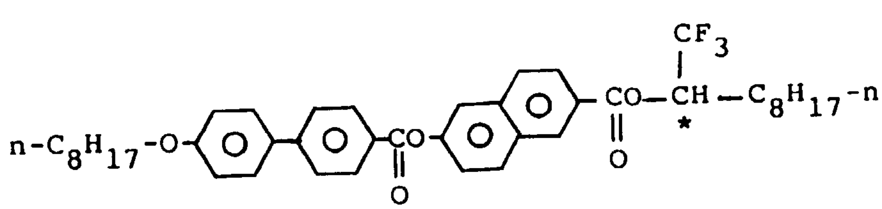

Example 1 was repeated except that optically active 1-trifluoromethylnonyl alcohol (2.3 g) was used in place of the optically active 1-trifluoromethylheptyl alcohol (2.2 g) used in (1) of Example 1 to obtain the titled compound (0.44 g) having specific rotation $[\alpha]_D^{20}$ = +44.2°.

Phase transition temperatures (°C) which were observed under a polarizing microscope using a hot stage were

as follows.

Cry ← S*(3) ← S*c ← SA ← Iso
84 133 135.6 163.8

where S*(3): optically tristable state liquid crystal phase.

IR spectrum (KBr) of the titled compound is shown in Fig. 6.

Example 3 (process B)

(1) Synthesis of 4-n-undecanoyloxy-4'-hydroxybiphenyl

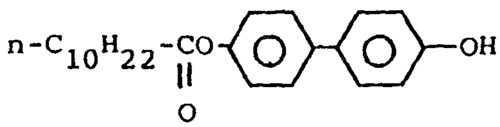

To a solution of 4-methylcarbonyloxy-4'-hydroxybiphenyl (2.3 g) and triethylamine (1.1 g) in methylene chloride (50 ml) was gradually added dropwise undecanoyl chloride (n-$C_{10}H_{21}COCl$) (2.1 g). Dimethylaminopyridine (0.3 g) was further added thereto and the mixture was stirred for 24 hours. After the solvent was distilled off, the residue was added to tetrahydrofuran containing tert-butylamine and the mixture was refluxed at 70°C for 2 hours and hydrolyzed. After the solvent was distilled off, dilute hydrochloric acid was added to the residue to render the solution neutral and the solution was extracted with diethyl ether. After the solvent was distilled off, the residue was washed with hexane and recrystallized from water/ethanol (1/9) solution to obtain the titled compound (about 1.4 g).

(2) Synthesis of 1-trifluoromethylheptyl 6-[4-(n-undecanoyloxy)biphenyl-4'-oxycarbonyl]naphthalene-2-carboxylate

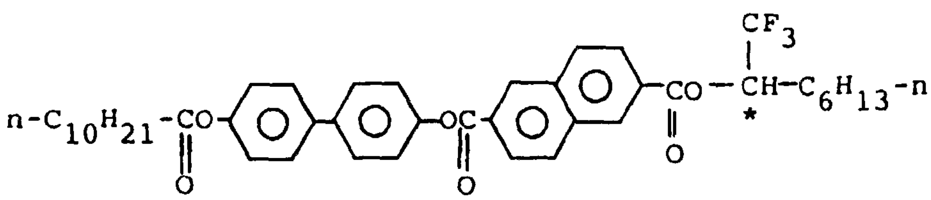

To a solution of 4-n-undecanoyloxy-4'-hydroxybiphenyl (0.6 g) obtained in (1) above and triethylamine (0.11 g) in methylene chloride (40 ml) was added gradually dropwise a solution of 6-(1-trifluoromethylheptyloxycarbonyl)naphthalene-2-carboxylic acid chloride (0.7 g) obtained in (1) of Example 3 in a small amount of methylene chloride. Thereafter, dimethylaminopyridine (0.03 g) was added thereto and the mixture was stirred at room temperature for 24 hours.

After the solution was made neutral with dilute hydrochloric acid, the methylene chloride layer was extracted and dried over anhydrous magnesium sulfate. The solvent was distilled off and the residue was purified by silica gel column chromatography (developer: hexane/ethyl acetate = 20/1) to obtain the titled compound (0.44 g). Phase transition temperatures (°C) of the titled compound which were observed under a polarizing microscope using a hot stage were as follows.

Cry ← S*(3) ← S*c ← SA ← Iso
75 120 156.9 184.2

where S*(3): optically tristable state liquid crystal phase

The novel liquid crystals of the present invention have bistable states and tristable states and have a wide variety of applications such as display and switching devices.

## Claims

**1.** A liquid crystal compound which is represented by the formula [I]:

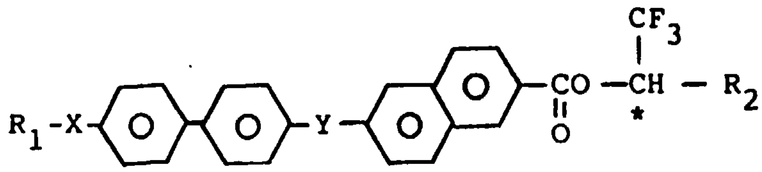

....(I)

wherein $R_1$ represents an alkyl group of 5-18 carbon atoms; $R_2$ represents an alkyl group of 6-16 carbon atoms; X represents a -O- or

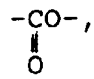

Y represents

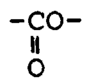 or 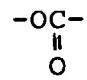

and when X is O Y is

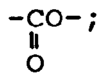

and * indicates an optically active center.

**2.** A liquid crystal compound represented by the formula [II]:

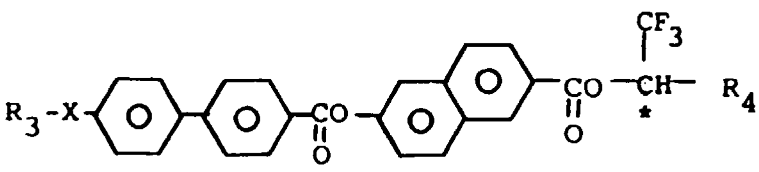

....(II)

wherein $R_3$ represents an alkyl group of 8-18 carbon atoms; $R_4$ represents an alkyl group of 6-14 carbon atoms; X represents a -O- or

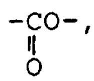

and * indicates an optically active center.

**Patentansprüche**

**1.** Flüssigkristall-Verbindung, wiedergegeben durch die Formel [I],

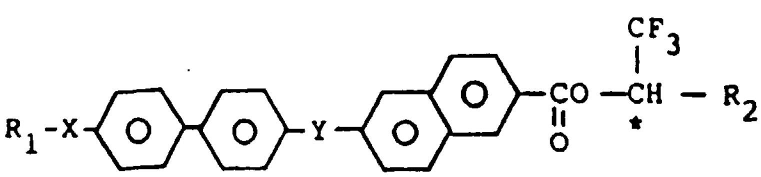

[I]

worin

$R_1$ für eine Alkyl-Gruppe mit 5 bis 18 Kohlenstoff-Atomen steht;
$R_2$ für eine Alkyl-Gruppe mit 6 bis 16 Kohlenstoff-Atomen steht,
X für -O- oder

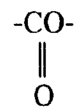

steht;
Y für

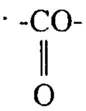 oder 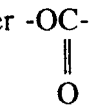

steht; und
Y für

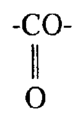

steht, wenn X für -O- steht; und
* ein optisch aktives Zentrum angibt.

**2.** Flüssigkristall-Verbindung, wiedergegeben durch die Formel [II],

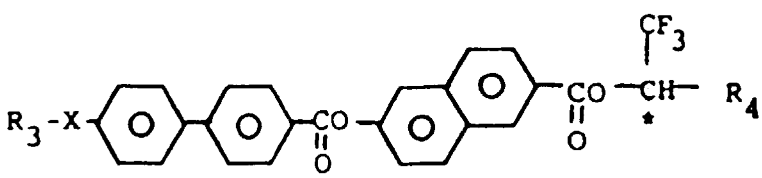 [II]

worin

$R_3$ für eine Alkyl-Gruppe mit 8 bis 18 Kohlenstoff-Atomen steht;
$R_4$ für eine Alkyl-Gruppe mit 6 bis 14 Kohlenstoff-Atomen steht,
X für -O- oder

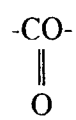

steht; und
* ein optisch aktives Zentrum angibt.

## Revendications

**1.** Cristal liquide qui est représenté par la formule [I]

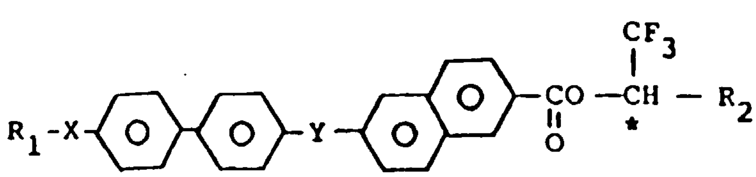 ....[I]

dans laquelle $R_1$ représente un groupe alcoyle de 5 à 18 atomes de carbone et $R_2$ représente un groupe alcoyle de 6 à 16 atomes de carbone; X représente un -O- ou

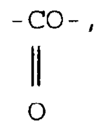

Y représente

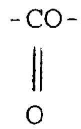 ou 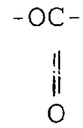

et quand X est O, Y est

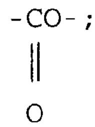

et * indique un centre optiquement actif.

**2.** Cristal liquide représenté par la formule [II]

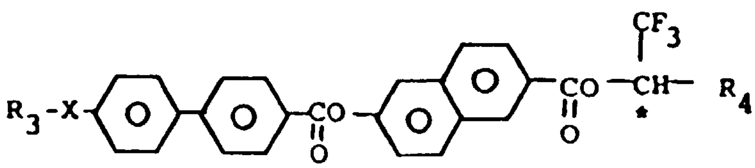 ....(II)

dans laquelle $R_3$ représente un groupe alcoyle de 8 à 18 atomes de carbone, $R_4$ représente un groupe alcoyle de 6 à 14 atomes de carbone; X représente un -O- ou un

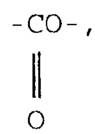

et * indique un centre optiquement actif.

FIG. 1

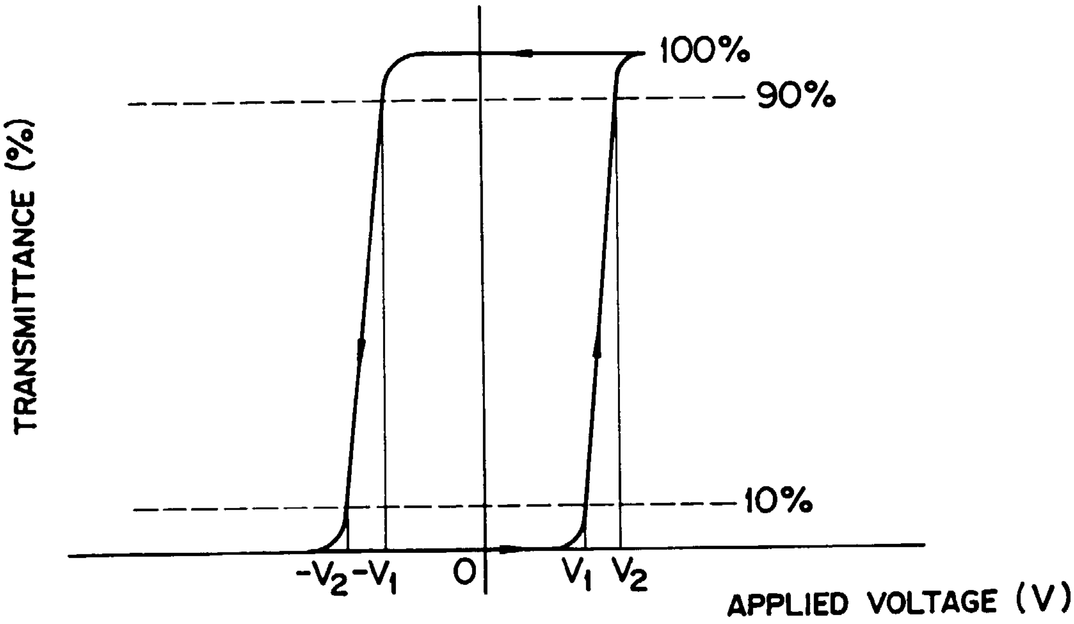
100%
90%
10%
TRANSMITTANCE (%)
-V2 -V1
0
V1 V2
APPLIED VOLTAGE (V)

FIG. 2

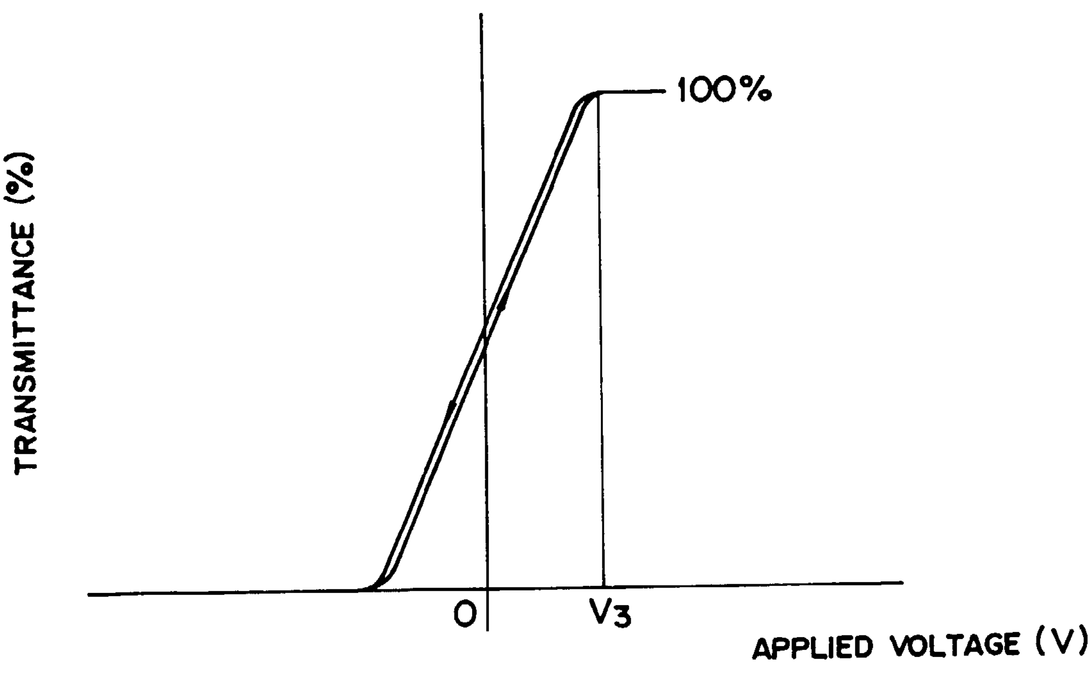
100%
TRANSMITTANCE (%)
0
V3
APPLIED VOLTAGE (V)

FIG. 3

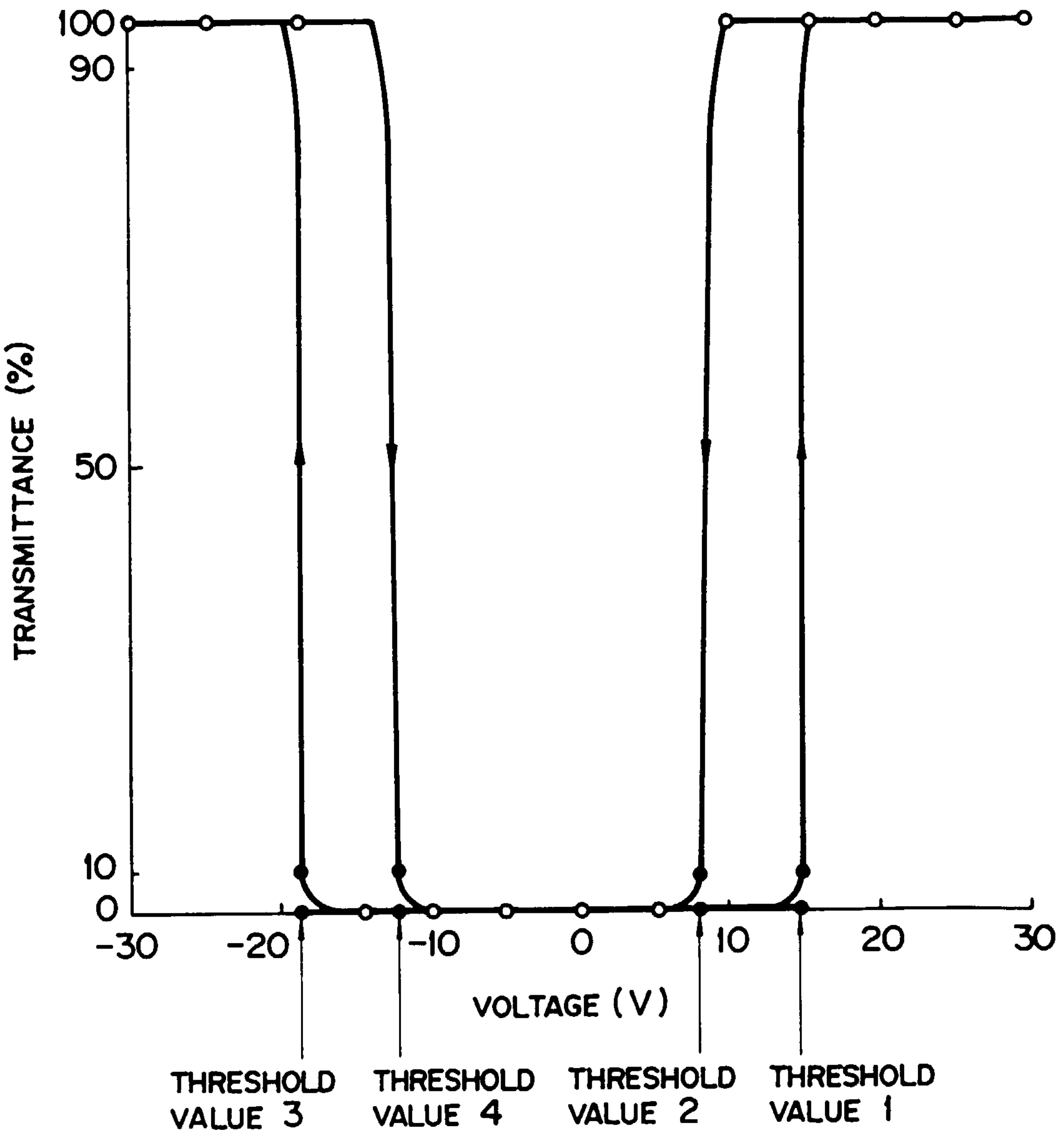
100
90
50
10
0
TRANSMITTANCE (%)
-30
-20
-10
0
10
20
30
VOLTAGE (V)
THRESHOLD VALUE 3
THRESHOLD VALUE 4
THRESHOLD VALUE 2
THRESHOLD VALUE 1

FIG. 4

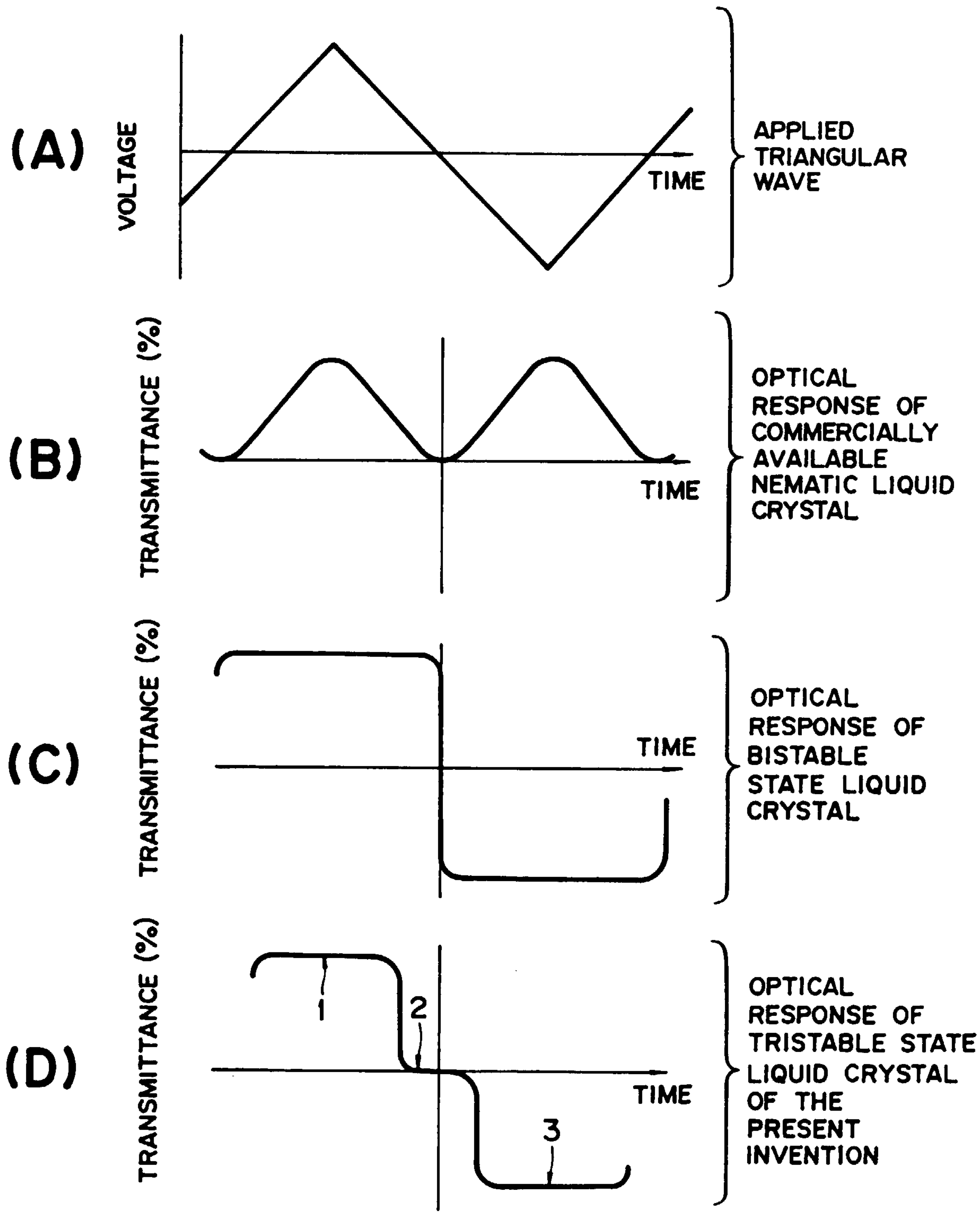
(A)
VOLTAGE
TIME
APPLIED TRIANGULAR WAVE
(B)
TRANSMITTANCE (%)
TIME
OPTICAL RESPONSE OF COMMERCIALLY AVAILABLE NEMATIC LIQUID CRYSTAL
(C)
TRANSMITTANCE (%)
TIME
OPTICAL RESPONSE OF BISTABLE STATE LIQUID CRYSTAL
(D)
TRANSMITTANCE (%)
1
2
3
TIME
OPTICAL RESPONSE OF TRISTABLE STATE LIQUID CRYSTAL OF THE PRESENT INVENTION

FIG. 5

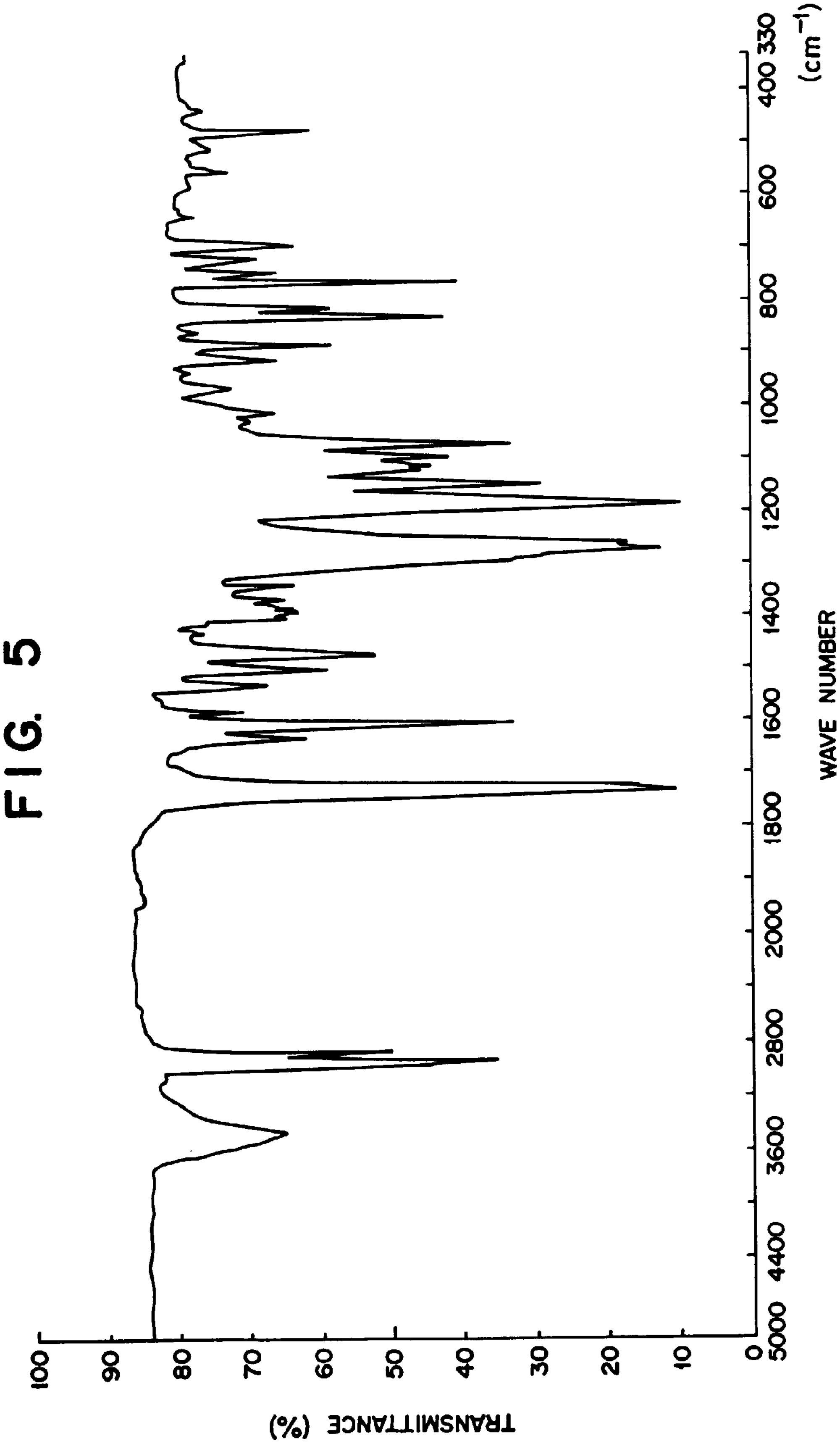
TRANSMITTANCE (%)
100
90
80
70
60
50
40
30
20
10
0
5000
4400
3600
2800
2000
1800
1600
1400
1200
1000
800
600
400
330
(cm$^{-1}$)
WAVE NUMBER

FIG. 6

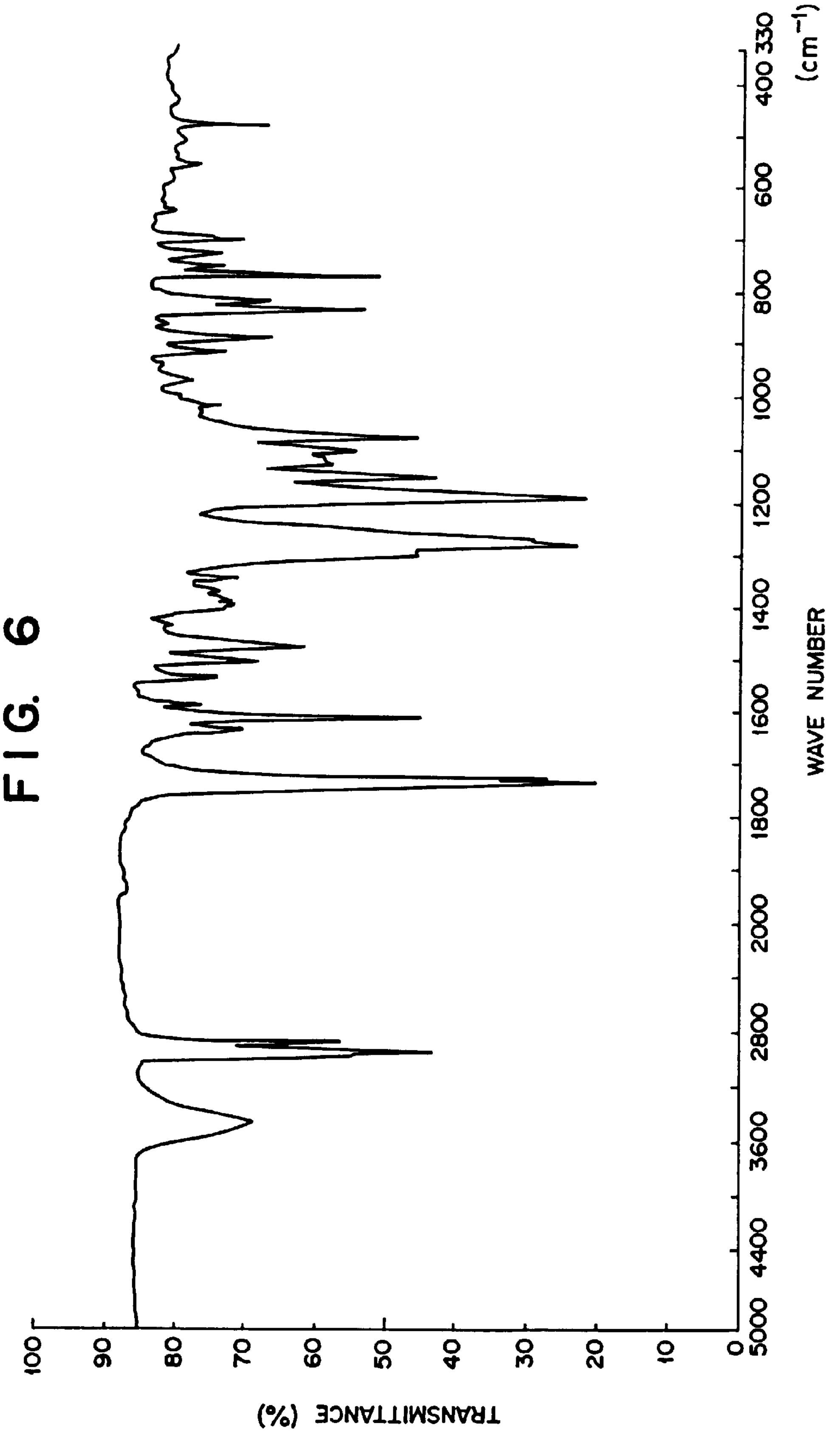
TRANSMITTANCE (%)
100
90
80
70
60
50
40
30
20
10
0
5000
4400
3600
2800
2000
1800
1600
1400
1200
1000
800
600
400
330
(cm$^{-1}$)
WAVE NUMBER